# EUROPÄISCHE PATENTSCHRIFT

(11) **EP 1 681 078 B1**
(45) Veröffentlichungstag und Bekanntmachung des Hinweises auf die Patenterteilung: **24.12.2008**
(21) Anmeldenummer: 06100185.5
(22) Anmeldetag: 10.01.2006
(51) Int. Cl.: A61Q 5/06, A61K 8/81

(54) **Haarsprayformeln mit hohen VOC-Anteilen und mit einem Terpolymer aus Methacrylsäure, Methylmethacrylat, Hydroxyethylmethacrylat und Butylacrylat**
Hairspray formulation with high VOC and a terpolymer of methacrylic acid, methyl methacrylate, hydroxyethyl methacrylate and butyl acrylate
Laque capillaire en aérosol avec un haut teneur des composés organiques volatils et un terpolymère d'acide méthacrylique, méthylméthacrylique, hydroxyéthylméthacrylique et butylméthacrylique

(30) Priorität: 13.01.2005 DE 102005001820
(43) Veröffentlichungstag der Anmeldung: 19.07.2006
(73) Patentinhaber: Beiersdorf AG, 20245 Hamburg (DE)
(72) Erfinder: Detert, Marion, 22455 Hamburg (DE); Dingler, Christian, 22527 Hamburg (DE); Pilzner, Anke, 22459 Hamburg (DE)

(56) Entgegenhaltungen:
- EP-A- 0 705 595
- DE-A1- 10 033 022
- US-A1- 2001 003 581
- ANDREA KEENAN OF THE ROHM AND HAAS COMPANY: "Hair styling formulations containing Acudyne 180 hair fixative polymer and Aculyn rheology modifiers" RESEARCH DISCLOSURE, MASON PUBLICATIONS, HAMPSHIRE, GB, Bd. 478, Nr. 8, Februar 2004 (2004-02), XP007133362 ISSN: 0374-4353

## Beschreibung

Zur temporären Verformung und Stabilisierung der Frisur werden Produkte eingesetzt, die als Stylingmittel bekannt sind. Hierzu zählen u. a. auch Haarfestiger oder Festigerschäume.

Üblicherweise bestehen diese Mittel zur Festigung der Haare aus alkoholischen oder wässrig alkoholischen Lösungen von filmbildenden natürlichen oder synthetischen Polymeren. Diese Polymere können aus der Gruppe der nichtionischen, kationischen, amphotern oder anionischen Polymere ausgewählt werden.

Haarfestiger können als Aerosol oder als Sprühprodukt auf die Haare aufgetragen werden.

Als festigende Polymere werden bevorzugt anionische oder amphotere Polymere eingesetzt, da diese eine sehr gute Festigungsleistung besitzen aber auch besonders harte und spröde Film bilden. Somit sind die Pflegeleistungen des Produktes, wie z. B. ein guter Griff des Haares oder eine gute und leichte Auskämmbarkeit des Produktes nicht gegeben.

VOC sind flüchtige organische Verbindungen. VOC im Sinne der vorliegenden Schrift sind flüchtige Kohlenwasserstoffe (Propan, Butan, i-Butan, Pentan, usw.), flüchtige Ether wie Dimethylether und flüchtige Alkohole wie Methanol oder Ethanol.

Das Dokument DE 69529003 offenbart Zubereitungen mit einer großen Zahl von Acrylatcopolymeren, jedoch keine erfindungsgemäßen. Insbesondere weisen die dort offenbarten Zubereitungen nicht hohe Anteile an VOC auf.

Das Dokument DE 10033022 offenbart kosmetische und/oder pharmazeutische Zubereitungen, enthaltend bestimmte Dialkylcarbonate und Teibmittel, jedoch keine, die Acrylatpolymere enthalten.

Das Dokument DE 69526896 offenbart Haarstylingzubereitungen Haarstylingzubereitungen mit Acrylharzen und höchstens 70 Gew.-% VOC.

Das Dokument US 6368575 offenbart Haarstylingzubereitungen mit Acrylharzen und höchstens 70 Gew.-% VOC, die sich durch besonders geringe Korrosivität auszeichnen.

Das Dokument DE 10107216 offenbart kosmetische Mittel, enthaltend eine Wirkstoffkombination aus bestimmten Kohlenwasserstoffen und einem bestimmten kosmetischen Öl. Diese Mittel zeichnen sich durch eine leichte Formulierbarkeit und gute Wirksamkeit sowie gute biologische Abbaubarkeit aus, enthalten jedoch keine Acrylatpolymere.

Das Dokument EP 705595 A2 offenbart wässrige Haargestaltungszubereitungen, die bestimmte Polymere enthalten können, aber nicht mehr als 70 Gew.%, bevorzugt nicht mehr als 50 Gew.-% flüchtiger organischer Verbindungen enthalten. Über Haargestaltungszubereitungen mit höheren Gehalten an flüchtigen, organischen Verbindungen wird nichts offenbart.

Es wurde nun überraschenderweise festgestellt, dass ein Haarspray mit einem Gehalt an flüchtigen organischen Verbindungen von 75 bis 99 Gew.-% enthaltend ein Terpolymer aus Methacrylsäure, Methylmethacrylat, Hydroxyethylmethacrylat und Butylacrylat den Nachteilen des Standes der Technik abhilft. Das Haar fühlt sich gepflegt an und das Produkt lässt sich leicht aus den Haaren auskämmen.

Weiterhin ist es erfindungsgemäß bevorzugt, wenn das Gewichtsmittel des Molekulargewichtes Terpolymers 81000 bis 99000 g/mol beträgt.

Außerdem ist es erfindungsgemäß bevorzugt, wenn der Gehalt an flüchtigen organischen Verbindungen 81 bis 99 Gew.-%, besonders bevorzugt 83 bis 99 Gew.-% beträgt.

Weiter ist es bevorzugt, wenn das erfindungsgemäße Terpolymer aus 18 Gew.-% Methacrylsäure, 47 Gew.-% Methylmethacrylat, 10 Gew.-% Hydroxyethylmethacrylat und 25 Gew.-% Butylacrylat hergestellt wurde.

Darüber hinaus ist es bevorzugt, wenn das erfindungsgemäße Haarspray frei von Silikonölen ist.

Ausserdem ist es bevorzugt, wenn der Gehalt an erfindungsgemäßem Terpolymer 0,1 bis 10 Gew.-%, besonders bevorzugt 1 bis 4 Gew.-% beträgt.

Weiter ist es bevorzugt, wenn die Glasübergangstemperatur des erfindungsgemäßen Terpolymers 81 bis 105°C, besonders bevorzugt 85 bis 95°C beträgt.

Außerdem ist es bevorzugt, wenn das erfindungsgemäße Haarspray zusätzlich 0,01 bis 5 Gew.%, besonders bevorzugt 0,1 bis 0,2 Gew.-% eines Dialkylcarbonates, besonders bevorzugt Dioctylcarbonat enthält.

Die Erfindung umfasst auch die Verwendung von erfindungsgemäßem Terpolymer in Kombination mit Dialkylcarbonat nach einem der vorangehenden Ansprüche in Haarsprays zur Verbesserung des Griffs und/oder der Kämmbarkeit des behandelten Haares, sowie der Auskämmbarkeit des Produktes aus dem Haar.

Bevorzugt wird als erfindungsgemäß ganz besonders bevorzugtes Terpolymer das Acudyne 180 (proposed INCI: Acrylates/Hydroxyacrylates Copolymer) von der Firma Rohm & Haas eingesetzt. Als erfindungsgemäß ganz besonders bevorzugtes Dialkylcarbonat wird bevorzugt das Cetiol CC (INCI: Dioctyl Carbonate) von der Firma Cognis eingesetzt.

Insbesondere diese Kombination führt dazu, dass der Haarfestiger, der ein Sprühprodukt oder ein Aerosol sein kann, neben den guten Stylingeigenschaften das Haar mit einem guten Griff und einer guten Kämmbarkeit versorgt. Das Haar wirkt gepflegt und das Produkt lässt sich leicht aus den Haaren auskämmen.

Daher richtet sich diese Erfindung auch auf die Verwendung von Dialkylcarbonaten zur Verbesserung der Eigenschaften von Acrylatcopolymeren auf dem Haar.

Weiterhin ist es bevorzugt, wenn erfindungsgemäße Haarsprays 0 - 55 % eines Treibmittels enthalten.

Natürlich können neben dem bevorzugten Terpolymer weitere Polymere zur Festigung eingesetzt werden.

Geeignete amphotere Polymere sind Octylacrylamide/Acrylates/Butylaminoethyl Methacrylate Copolymer (Amphomer 28-4910; National Starch), Acrylates/Lauryl Acrylate/Stearyl Acrylate/Ethylamine Oxide Methacrylate Copolymer (Diaformer; Mitsubishi) oder Methacryloyl Ethylbetaine/Methacrylates Copolymer (Diaformer; Mitsubishi). Weiterhin sind geeignet Copolymerisate aus Carboxylgruppen oder Sulfongruppen enthaltenden Monomeren, z. B. (Meth)Acrylsäure und Itaconsäure mit basischen insbesondere Aminogruppen enthaltenden Monomeren wie z. B. Mono- bzw. Dialkylamino-alkyl(meth)acrylaten und/oder Mono- bzw. Dialkylaminoalkyl(meth)acrylamiden, Copolymere aus N-Octylacrylamid, Methylmethacrylat, Hydroxypropylmethacrylat, N-tert.-Butylaminoethylmethacrylat und Acrylsäure.

Geeignete anionische Polymere sind Homo- oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbare-Verbindungen, welche mindestens eine Säuregruppe besitzen., insbesondere Carboxyvinylmonomere. Geeignete Säuregruppen enthaltende Monomere sind beispielsweise Acrylsäure, Meth-acrylsäure, Crotonsäure, Maleinsäure bzw. Maleinsäureanhydrid oder deren Monoester, Aldehydcarbonsäuren oder Ketocarbonsäuren.

Geeignete Polymere sind: INCI: VA/Crotonates/Vinylneodecanoate Copolymer z. B. Resyn 28-2930 von National Starch; INCI: Acrylates Copolymer, z.B. Luvimer von BASF; INCI: Sodium Polystyrene Sulfonate z. B. Flexan 130 von National Starch.

Geeignete anionische Polymere sind auch in Wasser lösliche oder dispergierbare anionische Polyurethane, z. B. Luviset PUR von BASF oder Polyester.

Als Neutralisationsmittel für Polymere die Säuregruppen enthalten können folgende Stoffe eingesetzt werden:
Hydroxide, deren Kation ein Ammonium oder ein Alkalimetall ist wie z. B. NaOH oder KOH.

Andere Neutralisationmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), 2-Amino-1,3-propandiol, Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM), und Dimethyl Stearamin (DMS).

Geeignete Polymere mit basischen Gruppen sind z. B. Copolymere von aminsubstituierten Vinylmonomeren und nicht aminsubstituierten, nicht kationischen Monomeren. Geeignete kationische Polymere sind: INCI: Polyquaternium-11, z.B. Gafquat 755 von ISP oder Luviquat PQ-11 von BASF; INCI: Polyquaternium-55, z. B. Styleze W-20 von ISP; INCI: Polyquaternium-16, z. B. die Luviquat Typen FC 370, FC 550, FC 905 oder HM552 von BASF; INCI: Polyquaternium-46, z. B. Luviquat Hold von BASF.

Geeignete nichtionische Polymere sind z. B. Homopolymere des Vinylpyrrolidons, z. B. Luviskol K von BASF oder Homopolymere des N-Vinylformamids z. B. PVF von National Starch.

Weitere geeignete Polymere sind Copolymereisate aus Vinylpyrrolidon und Vinylacetat z. B. Luviskol VA Typen von BASF, Terpolymere aus Vinylpyrrolidon, Vinylacetat und Propionat wie z. B. Luviskol VAP von BASF, Polyvinylcaprolactam, Polyvinylamide und deren Salze sowie Copolymere aus Vinylpyrrolidon und Dimethylaminoethylmethacrylat, Terpolymere aus Vinylcaprolactam, Vinylpyrrolidon und Dimethylaminomethacrylat; Polysiloxane und dergleichen mehr.

In den erfindungsgemäßen Stylingformulierungen können als Pflegestoffe bevorzugt cyclische Polydimethylsiloxane (Cyclomethicone) in Konzentrationen von z. B. 0.1 - 1.0 Gew.-% der Gesamtformulierung oder Silikontenside (Polyethermodifizierte Siloxane) vom Typ Dimethicone Copolyol z. B. in Konz. 0.01 - 1.0 Gew.-% des Gesamtgewichts der Zubereitung bevorzugt zum Einsatz kommen. Cyclomethicone werden u. a. unter der Handelsbezeichnungen Abil K4 von der Gesellschaft Goldschmidt oder z.B. DC 244, DC 245 oder DC 345 von der Gesellschaft Dow Corning angeboten. Dimethicone Copolyole werden z. B. unter der Handelsbezeichnung DC 193 von der Gesellschaft Dow Corning bzw. Belsil DM 6031 von der Gesellschaft Wacker angeboten.

Optionale herkömmliche Additive können ebenfalls in die Formulierung einbezogen sein, um der Zusammensetzung bestimmte Modifizierungseigenschaften zu verleihen: dies sind Silikone oder Silikonderivate, Benetzungsmittel, Feuchthaltemittel, Weichmacher wie Glycerin, Glykol und Phthalester und - ether, erweichende Mittel, Riechstoffe und Parfüms, UV, Absorber, Farbstoffe , Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, Emulgatoren, oberflächenaktive Mittel, Viskositätsmodifizierer, Verdicker und Rheologiemodifizierer, Geliermittel, Trübungsmittel, Stabilisatoren, Sequestierungsmittel, Komplexbildner, Perlglanzmittel, ästhetische Verstärker, Fettsäuren, Fettalkohole, Triglyceride, botanische Extrakte, Klärhilfsmittel und Filmbildner.

Diese Additive sind im allgemeinen in einer Konzentration von etwa 0,01 % bis 10 Gew.-% bezogen auf das Gesamtgewicht der Zusammensetzung vorhanden

Die Formulierungen können Treibgase enthalten. Geeignete Treibmittel für Aerosole sind:
Kohlenwasserstoffe wie Butan, Isobutan und Propan. Bevorzugt in Haarsprays werden auch eingesetzt Dimethylether oder Fluorkohlenwasserstoffe wie z. B. 1,2-diflourethan (Treibmittel 152A).

Andere Treibmittel sind Stickstoff, Kohlendioxid, Distickstoffoxid oder komprimierte Luft.

Bevorzugte Lösungsmittel, die in den Formulierungen enthalten sind, sind neben Wasser, organische Lösungsmittel wie aliphatische Alkohole mit C1-4 Kohlenstoffatomen oder Gemische daraus. Es können auch andere Lösungsmittel eingesetzt werden wie unverzweigte oder verzweigte Kohlenwasserstoffe wie Pentan, Hexan, Isopentan und zyklische Kohlenwasserstoffe wie Cyclopentan und Cyclohexan.

Mögliche wasserlösliche Lösungsmittel sind Glycerin, Ethylenglycol und Propylenglycol.

Des Weiteren können als Additive Dialkylcarbonate der Formel (I):

R¹O(CH₂ CH₂ O)ₙ (C=O)(OCH₂ CH₂)ₘ OR² (I)

eingesetzt werden, die die Pflegeeigenschaften des Produktes verbessern. Dialkylcarbonate stellen grundsätzlich bekannte Stoffe dar, auch wenn einige der beanspruchten Carbonate an dieser Stelle erstmals beschrieben werden. Grundsätzlich lassen sich die Stoffe durch die Umesterung von Dimethyl- oder Diethylcarbonat mit den genannten Hydroxyverbindungen nach den Verfahren des Stands der Technik herstellen; eine Übersicht hierzu findet sich beispielsweise in Chem.Rev. 96, 951 (1996). Dialkylcarbonate der Formel (I), die sich in besonderer Weise zur Lösung der vorliegenden Aufgabenstellung eignen, erfüllen eine der folgenden Bedingungen:
(1) R1 steht für einen linearen Alkylrest mit 6 bis 18 und insbesondere 8 bis 10 Kohlenstoffatomen, oder einen 2-Ethylhexylrest und R2 für R1 oder Methyl;
(2) R1 steht für einen linearen Alkylrest mit 12 bis 18 Kohlenstoffatomen, R2 für R1 oder Methyl und n und m jeweils für Zahlen von 1 bis 10;
(3) R1 steht für einen Rest eines Polyols, welches ausgewählt ist aus der Gruppe, die gebildet wird von Glycerin, Alkylenglycolen, technischen Oligoglyceringemischen, Methylolverbindungen, Niedrigalkylglucosiden, Zuckeralkoholen, Zuckern und Aminozuckern, und R2 für R1 einen linearen oder verzweigten Alkylrest mit 8 bis 12 Kohlenstoffatomen oder Methyl.

Typische Beispiele für Dialkylcarbonate der beiden Gruppen (1) und (2) sind vollständig oder partielle Umesterungsprodukte von Dimethyl- und / oder Diethylcarbonat mit Capronalkohol, Caprylalkohol, 2- Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linoiylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylesteren auf Basis von Fetten und Ölen oder Aldehyden aus der Roelenschen Oxosynthese sowie als Monomerfraktion bei der Dimerisierung von ungesättigten Fettalkoholen anfallen. In gleicher Weise geeignet sind die Umesterungsprodukte der niederen Carbonate mit den genannten Alkoholen in Form ihrer Addukte mit 1 bis 100, vorzugsweise 2 bis 50 und insbesondere 5 bis 20 Mol Ethylenoxid, vorzugsweise kommen Di-n-octylcarbonate in Frage. Vorzugsweise werden Dioctyl- oder Dihexylcarbonate verwendet.

Die Carbonate der Gruppe (3) werden hier erstmals beschrieben. Es handelt sich um Stoffe, die erhalten werden, indem man Dimethyl- und/oder Diethylcarbonat ganz oder partiell mit Polyolen umestert. Polyole, die im Sinne der Erfindung in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Propylenglycol, Butylenglycol, Hexylenglycol, sowie Polyethylenglycole mit einem durchschnittlichen Molekulargewicht von 100 bis 1000 Dalton.;
- Technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit;
- Zucker mit bis 5 bis 12 Kohlenstoffatomen wie beispielsweise Glucose oder Saccharose;
- Aminozucker wie beispielsweise Glucamin.

Bei dieser Reaktion kann es natürlich nicht nur zum Austausch einer Methyl- oder Ethyigruppe gegen einen Polyolrest kommen, vielmehr wird ein Gemisch erhalten, bei den mehrere oder sogar alle Hydroxylgruppen des Polyols mit Carbonatgruppen verknüpft sind, so dass sich gegebenenfalls sogar eine oligomere bzw. polymere Netzstruktur ergibt. Stoffe dieser Art sollen im Sinne der Erfindung ebenfalls durch die allgemeine Formel (I) umfasst werden.

Die erfindungsgemäßen Mittel können die Dialkylcarbonate, vorzugsweise Dioctyl- und / oder Dihexylcarbonat und insbesondere Di-n-Octylcarbonat in Mengen von 0 bis 10, vorzugsweise 0,01 bis 5 und insbesondere 0,01 bis 2 Gew.-% - bezogen auf die Endzusammensetzung - enthalten.

### Beispiele

Folgende Produkte wurden eingesetzt:
Acrylates/Hydroxyacrylates Copolymer: Acudyne 180, Fa. Rohm and Haas,
Octylacrylamide/ Acrylates/ Butylaminoethyl Methacrylate Copolymer: Amphomer, Fa. National Starch,
Aminomethylpropanol: Aminomethylpropanol 95, Fa. Angus,
Dialkylcarbonat: Dicaprylylcarbonat, Cetiol CC, Fa. Cognis,
Dimethylether, Demeon D, Akzo Nobel,
n-Butan, Drivosol 12, Fa. Degussa,
Polyurethane-14 AMP-Acrylates Copolymer: DynamX, Fa. National Starch,
Silikonöl: Peg-12 Dimethicone, Dow Corning 193 Surfactant, Fa. Dow Corning.

### Haarfestiger

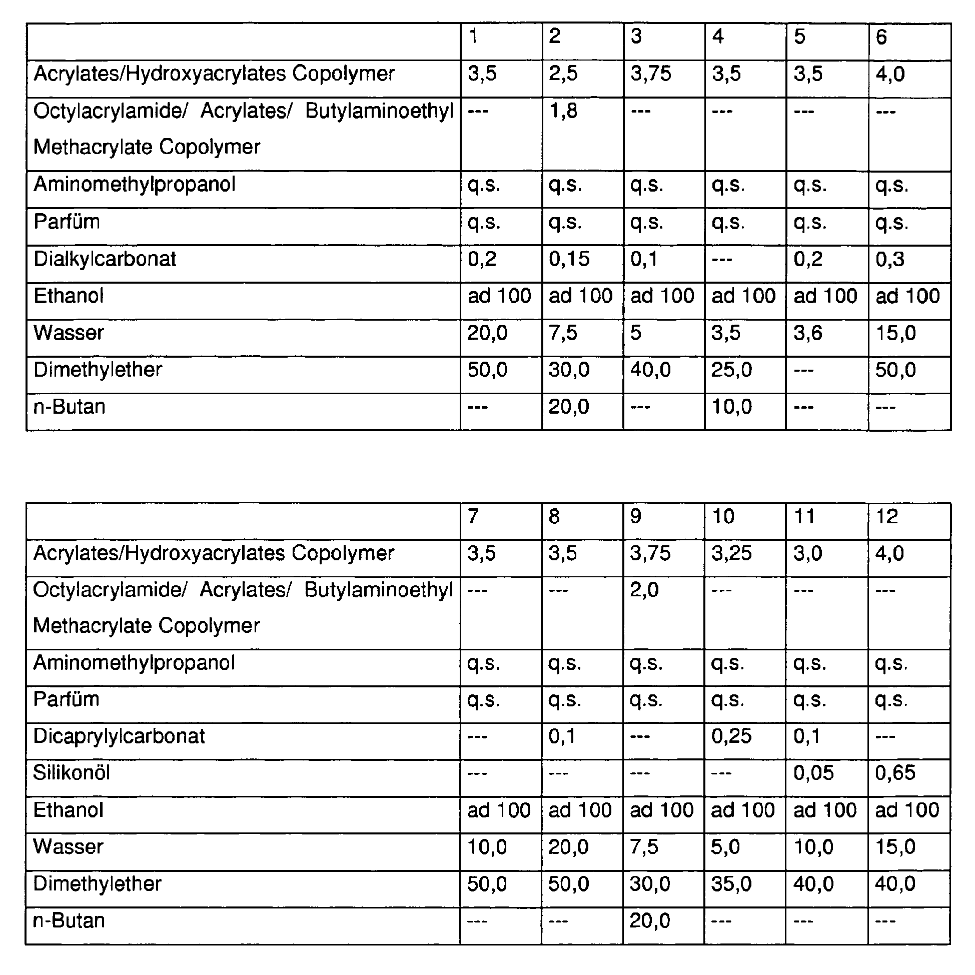

## Patentansprüche

1. Haarspray mit einem Gehalt an flüchtigen organischen Verbindungen von 75 bis 99 Gew.-% enthaltend ein Terpolymer aus Methacrylsäure, Methylmethacrylat, Hydroxyethylmethacrylat und Butylacrylat.

2. Haarspray nach Anspruch 1 **dadurch gekennzeichnet, dass** das Gewichtsmittel des Molekulargewichtes Terpolymers 81000 bis 99000 g/mol beträgt.

3. Haarspray nach einem der vorangehenden Ansprüche **dadurch gekennzeichnet, dass** der Gehalt an flüchtigen organischen Verbindungen 81 bis 99 Gew.-% beträgt.

4. Haarspray nach einem der vorangehenden Ansprüche **dadurch gekennzeichnet, dass** das Terpolymer aus 18 Gew.-% Methacrylsäure, 47 Gew.-% Methylmethacrylat, 10 Gew.-% Hydroxyethylmethacrylat und 25 Gew.-% Butylacrylat hergestellt wurde.

5. Haarspray nach einem der vorangehenden Ansprüche **dadurch gekennzeichnet, dass** es frei von Silikonölen ist.

6. Haarspray nach einem der vorangehenden Ansprüche **dadurch gekennzeichnet, dass** der Gehalt an Terpolymer 0,1 bis 10 Gew.-% beträgt.

7. Haarspray nach einem der vorangehenden Ansprüche **dadurch gekennzeichnet, dass** die Glasübergangstemperatur des Terpolymers 81 bis 105 °C beträgt.

8. Haarspray nach einem der vorangehenden Ansprüche **dadurch gekennzeichnet, dass** es zusätzlich 0,01 bis 5 Gew.% eines Dialkylcarbonates enthält.

9. Verwendung von Terpolymer in Kombination mit Dioctylcarbonat nach einem der vorangehenden Ansprüche in Haarsprays zur Verbesserung des Griffs und/oder der Kämmbarkeit des behandelten Haares, sowie der Auskämmbarkeit des Produktes aus dem Haar.

## Claims

1. Hairspray with a content of volatile organic compounds of from 75 to 99% by weight comprising a terpolymer of methacrylic acid, methyl methacrylate, hydroxyethyl methacrylate and butyl acrylate.

2. Hairspray according to Claim 1, **characterized in that** the weight average molecular weight of the terpolymer is from 81 000 to 99 000 g/mol.

3. Hairspray according to one of the preceding claims, **characterized in that** the content of volatile organic compounds is from 81 to 99% by weight.

4. Hairspray according to one of the preceding claims, **characterized in that** the terpolymer has been prepared from 18% by weight of methacrylic acid, 47% by weight of methyl methacrylate, 10% by weight of hydroxyethyl methacrylate and 25% by weight of butyl acrylate.

5. Hairspray according to one of the preceding claims, **characterized in that** it is free from silicone oils.

6. Hairspray according to one of the preceding claims, **characterized in that** the content of terpolymer is from 0.1 to 10% by weight.

7. Hairspray according to one of the preceding claims, **characterized in that** the glass transition temperature of the terpolymer is from 81 to 105°C.

8. Hairspray according to one of the preceding claims, **characterized in that** it additionally comprises from 0.01 to 5% by weight of a dialkyl carbonate.

9. Use of terpolymer in combination with dioctyl carbonate according to one of the preceding claims in hairsprays for improving the feel and/or the combability of the treated hair, and also the ability of the product to be combed out of the hair.

## Revendications

1. Laque capillaire en aérosol présentant une teneur en composés organiques volatils de 75 à 99 % en poids, comprenant un terpolymère de l'acide méthacrylique, du méthacrylate de méthyle, du méthacrylate d'hydroxyéthyle et de l'acrylate de butyle.

2. Laque capillaire en aérosol selon la revendication 1, **caractérisée en ce que** la moyenne en poids du poids moléculaire du terpolymère est de 81000 à 99000 g/mol.

3. Laque capillaire en aérosol selon l'une quelconque des revendications précédentes, **caractérisée en ce que** la teneur en composés organiques volatils est de 81 à 99 % en poids.

4. Laque capillaire en aérosol selon l'une quelconque des revendications précédentes, **caractérisée en ce que** le terpolymère a été préparé à partir de 18 % en poids d'acide méthacrylique, de 47 % en poids de méthacrylate de méthyle, de 10 % en poids de méthacrylate d'hydroxyéthyle et de 25 % en poids d'acrylate de butyle.

5. Laque capillaire en aérosol selon l'une quelconque des revendications précédentes, **caractérisée en ce qu'**elle est exempte d'huiles siliconées.

6. Laque capillaire en aérosol selon l'une quelconque des revendications précédentes, **caractérisée en ce que** la teneur en terpolymère est de 0,1 à 10 % en poids.

7. Laque capillaire en aérosol selon l'une quelconque des revendications précédentes, **caractérisée en ce que** la température de transition vitreuse du terpolymère est de 81 à 105°C.

8. Laque capillaire en aérosol selon l'une quelconque des revendications précédentes, **caractérisée en ce qu'**elle comprend en outre de 0,01 à 5 % en poids d'un carbonate de dialkyle.

9. Utilisation d'un terpolymère en combinaison avec du carbonate de dioctyle selon l'une quelconque des revendications précédentes dans des laques capillaires en aérosol pour l'amélioration du toucher et/ou de la facilité de peignage des cheveux traités, ainsi que de l'aptitude du produit à être éliminé des cheveux par peignage.
